# EUROPÄISCHE PATENTANMELDUNG

(11) **EP 4 559 424 A1**
(43) Veröffentlichungstag der Anmeldung: **28.05.2025**
(21) Anmeldenummer: 24212761.1
(22) Anmeldetag: 13.11.2024
(51) Int. Cl.: A61B 34/20, A61B 34/00, A61B 90/20, A61B 90/25, A61B 90/50, G02B 21/00, G02B 21/22

(54) **VERFOLGEN VON BEWEGTEN OBJEKTEN WÄHREND DER AUTO-KONFIGURATION EINES OPERATIONMIKROSKOPIESYSTEMS**

(30) Priorität: 15.11.2023 DE 102023131862
(71) Anmelder: Carl Zeiss Meditec AG, 07745 Jena (DE)
(72) Erfinder: PHILIPP, Markus, 73447 Oberkochen (DE); SAUR, Stefan, 73447 Oberkochen (DE); BACHER, Neal, 73447 Oberkochen (DE)
(74) Vertreter: Kraus & Lederer PartGmbB

(57) **Zusammenfassung**

Es werden Techniken offenbart, welche eine automatische Konfiguration von ein oder mehreren Komponenten eines Operationsmikroskopiesystems zur Abbildung eines bewegten Objekts (242) betreffen. Bei den beschriebenen Techniken erfolgt eine selektive Anpassung der initial bestimmten Soll-Konfiguration des Operationsmikroskopiesystems, wenn eine Bewegung des bewegten Objekts erkannt wird; dabei erfolgt diese selektive Anpassung dann, wenn die Bewegung innerhalb einer vorgegebenen räumlichen Schranke (551) und/oder einer vorgegebenen zeitlichen Schranke liegt wird.

## Beschreibung

### TECHNISCHES GEBIET

Verschiedene Beispiele der Offenbarung betreffen Techniken, um ein Operationsmikroskopiesystem zur Bildgebung eines Objekts automatisch zu konfigurieren. Verschiedene Beispiele der Offenbarung betreffen Techniken, um bei der Ansteuerung mindestens einer Komponente des Operationsmikroskopiesystems eine Bewegung des Objekts zu berücksichtigen.

### HINTERGRUND

Medizinische Operationsmikroskopiesysteme (auch als robotische Visualisierungssysteme oder chirurgische Visualisierungssysteme bezeichnet) mit einem robotischem Stativ zur Positionierung eines Mikroskops sind aus dem Stand der Technik bekannt, siehe z.B. DE 10 2022 100 626 A1.

Das robotische Stativ kann dabei manuell gesteuert werden. Es sind aber auch Techniken bekannt, bei denen das robotische Stativ automatisch gesteuert wird, z.B. um eine Auto-Zentrierung und/oder Auto-Fokussierung auf ein bestimmtes Objekt wie z.B. ein Operationsinstrument (auch als Operationsbesteck oder Operationstool bezeichnet) zu ermöglichen. Ein Benutzerbefehl löst dabei einen Positionierungsvorgang aus, bei denen das robotische Stativ und/oder eine Objektivoptik des Mikroskops angesteuert werden.

Solche Techniken zur automatischen Steuerung sind z.B. bekannt aus US 10,456,035 B2. Dort ist eine automatische Positionskorrektur beschrieben. Eine Abweichung der relativen Position eines Objekts kann korrigiert werden. Wenn eine automatische Positionskorrektur jedes Mal durchgeführt wird, wenn sich die Position eines Beobachtungsobjekts verändert, kann sich der Benutzer, der das Beobachtungsbild betrachtet, seltsam fühlen. Deshalb sind auch andere Betriebsmodi für die Positionskorrektur offenbart. Der bedingte Automatikmodus ist ein Modus, in dem eine Positionsabweichung unter der Bedingung korrigiert wird, dass eine Abweichung der relativen Position einen zulässigen Bereich überschreitet. Der spezifizierte Zeitkorrekturmodus ist ein Modus, in dem eine Positionsabweichung unter der Bedingung korrigiert wird, dass ein Befehl für eine Positionskorrektur durch den Benutzer eingegeben wird.

Es wurde beobachtet, dass bei solchen vorbekannten Techniken die Benutzererwartung betreffend die Auto-Konfiguration und die tatsächliche, vom Operationsmikroskopiesystem vorgenommene Änderungen diskrepant sein können.

### ZUSAMMENFASSUNG

Deshalb besteht ein Bedarf für verbesserte Techniken zur automatischen Ansteuerung von Operationsmikroskopiesystemen. Insbesondere besteht ein Bedarf für verbesserte Techniken im Zusammenhang mit einer automatischen Konfiguration des Operationsmikroskopiesystems zur Bildgebung eines Objekts.

Diese Aufgabe wird gelöst von den Merkmalen der unabhängigen Ansprüche. Die Merkmale der abhängigen Ansprüche definieren Ausführungsformen.

Nachfolgend werden Aspekte im Zusammenhang mit der automatischen Konfiguration eines Operationsmikroskopiesystems mit einem Stativ und einem vom robotischen Stativ getragenen Mikroskop beschrieben.

Das Stativ kann, als allgemeine Regel, robotisch oder zumindest teilweise robotisch ausgebildet sein. Es wäre auch denkbar, dass das Stativ nicht robotisch ausgebildet ist.

Das Operationsmikroskopiesystem wird dabei zur Bildgebung eines vorgegebenen Objekts konfiguriert. Das bedeutet, dass das vorgegebene Objekt im Gesichtsfeld einer Kamera, beispielsweise eine Mikroskopkamera des Mikroskops, auf eine bestimmte Art und Weise angeordnet ist.

Die automatische Konfiguration kann zum Beispiel eine Auto-Zentrierung an einem bestimmten Referenzpunkt umfassen. Der Referenzpunkt kann z.B. eine Spitze oder ein Zentrum eines Objekts sein. Der Referenzpunkt kann z.B. einen geometrischen Schwerpunkt oder Mittelpunkt oder einem Aktivitätszentrum mehrerer Objekte umfassen. Bei einer solchen Auto-Zentrierung des Gesichtsfelds ist der Referenzpunkt anschließend im Zentrum des Mikroskopbilds angeordnet. Dazu wird ein zumindest teilweise robotisches Stativ entsprechend angesteuert.

Die Konfiguration kann alternativ oder zusätzlich die Einstellung eines Fokus des Mikroskops auf einen solchen Referenzpunkt umfassen (Auto-Fokus). Beispielsweise können Fokuslinsen verfahren werden, sodass ein solcher Referenzpunkt in der Fokusebene platziert wird.

Alternativ oder zusätzlich kann auch eine bestimmte Orientierung eines Mikroskops des Operationsmikroskopiesystems in Bezug auf einen solchen Referenzpunkt eingestellt werden (Auto-Orientierung): Beispielsweise kann eine bestimmte Orientierung des Mikroskops eingestellt werden (d.h. die optische Achse kann rotatorisch positioniert werden). Es wäre z.B. ein Pivotieren denkbar, ohne dass das Mikroskop translatorisch bewegt wird.

Es kann auch eine kombinierte translatorische und rotatorische Bewegung ausgeführt werden (kombinierte Auto-Positionierung und Auto-Orientierung).

Alternativ oder zusätzlich kann auch ein Zoom des Mikroskops eingestellt werden, beispielsweise derart, dass ein bestimmtes Objekt oder mehrere Objekte bildschirmfüllend sichtbar sind (Auto-Zoom).

Mittels der voranstehenden Techniken ist es möglich, eine solche Auto-Konfiguration des Operationsmikroskopiesystems derart durchzuführen, dass Benutzererwartungen besonders gut erfüllt werden. Insbesondere kann derart eine nahtlose Benutzererfahrung bereitgestellt werden, die auch in Hoch-Stress-Situationen für den Operateur ein zuverlässiges Systemverhalten ermöglicht.

Dazu wird ein bewegtes Objekt, welches die Grundlage für die Auto-Konfiguration des Operationsmikroskopiesystems bildet, während der Auto-Konfiguration des Operationsmikroskopiesystems verfolgt (das wird auch oftmals als "tracking" bezeichnet). Das bedeutet, dass überprüft wird, ob sich die Position und/oder die Orientierung des Objekts gegenüber seiner initialen Position und/oder Orientierung verändert; das bedeutet, dass überprüft wird, ob sich das Objekt bewegt.

Es wird dann überprüft, ob die Bewegung ein oder mehrere Kriterien erfüllt. Es kann beispielsweise überprüft werden, ob die Position und/oder Orientierung innerhalb einer vorgegebenen räumlichen Schranke ändert. Es kann alternativ oder zusätzlich auch überprüft werden, ob sich die Position und/oder Orientierung innerhalb einer vorgegebenen zeitlichen Schranke ändert.

Verändert sich z.B. die Position außerhalb der zeitlichen Schranke, wird die im Zusammenhang mit der Auto-Konfiguration nicht berücksichtigt. Verändert sich z.B. die Orientierung außerhalb der zeitlichen Schranke, wird die im Zusammenhang mit der Auto-Konfiguration nicht berücksichtigt. Verändert sich z.B. die Position außerhalb der räumlichen Schranke, wird die im Zusammenhang mit der Auto-Konfiguration nicht berücksichtigt.

Es wird ein Computer-implementiertes Verfahren zum Steuern eines Operationsmikroskopiesystems offenbart. Das Operationsmikroskopiesystem umfasst ein Stativ, zum Beispiel ein robotisches oder teilweise robotisches Stativ. Das Operationsmikroskopiesystem umfasst auch ein Mikroskop, welches vom Stativ getragen wird. Das Mikroskop kann zum Beispiel eine Kamera zum Erfassen eines Mikroskopbilds aufweisen. Das Mikroskop kann zum Beispiel ein Stereo-Mikroskop sein.

Das Verfahren umfasst das Empfangen eines Benutzerbefehls. Der Benutzerbefehl fordert eine Konfiguration des Operationsmikroskopiesystems zur Bildgebung eines vorgegebenen Objekts an. Das Verfahren umfasst auch das initiale Bestimmen einer Soll-Konfiguration des Operationsmikroskopiesystems basierend auf dem Benutzerbefehl. Das Verfahren umfasst auch das Ansteuern von mindestens einer Komponente des Operationsmikroskopiesystems zum Erreichen der Soll-Konfiguration. Das Verfahren umfasst auch das Bestimmen, ob sich die Position und/oder die Orientierung des beweglichen Objekts innerhalb einer vorgegebenen zeitlichen Schranke und/oder innerhalb einer vorgegebenen räumlichen Schranke verändert. Dieses Bestimmen erfolgt in einem zeitlichen Zusammenhang mit dem Ansteuern der mindestens einen Komponente. Das Verfahren umfasst auch das Anpassen der Soll-Konfiguration, wenn sich die Position und/oder Orientierung des beweglichen Objekts innerhalb der vorgegebenen zeitlichen Schranke und/oder innerhalb der vorgegebenen räumlichen Schranke verändert.

Einer Datenverarbeitungseinrichtung zur Steuerung eines Operationsmikroskopiesystems wird offenbart. Die Datenverarbeitungseinrichtung umfasst einem Prozessor. Der Prozessor ist eingerichtet, um Programmcode aus einem Speicher zu laden und auszuführen. Das Ausführen des Programmcodes bewirkt, dass der Prozessor das obenstehend offenbarte Verfahren zum Steuern des Operationsmikroskopiesystems ausführt.

Es wird auch ein Operationsmikroskopiesystem mit einer solchen Datenverarbeitungseinrichtung offenbart.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

### KURZE BESCHREIBUNG DER FIGUREN

FIG. 1 illustriert schematisch ein Operationsmikroskopiesystem gemäß verschiedenen Beispielen.
FIG. 2 ist ein Flussdiagramm eines beispielhaften Verfahrens.
FIG. 3 illustriert schematisch eine Auto-Zentrierung eines Gesichtsfelds einer Kamera eines Operationsmikroskops auf die Spitze eines Operationsinstruments gemäß verschiedenen Beispielen.
FIG. 4 entspricht FIG. 3 und illustriert den Effekt einer Bewegung des Operationsinstruments im zeitlichen Kontext mit der Auto-Zentrierung.
FIG. 5 entspricht FIG. 4 und illustriert weiterhin eine räumliche Schranke.
FIG. 6 illustriert eine angepasste Auto-Zentrierung unter Berücksichtigung der räumlichen Schranke aus FIG. 5.
FIG. 7 illustriert schematisch eine zeitliche Schranke in Zusammenhang mit einer Anpassung einer Soll-Konfiguration bei einer automatischen Bewegung eines robotischen Stativs.
FIG. 8 illustriert schematisch eine zeitliche Schranke in Zusammenhang mit einer Anpassung einer Soll-Konfiguration bei einer automatischen Bewegung eines robotischen Stativs.

### DETAILLIERTE BESCHREIBUNG

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Die Figuren sind schematische Repräsentationen verschiedener Ausführungsformen der Erfindung. In den Figuren dargestellte Elemente sind nicht notwendigerweise maßstabsgetreu dargestellt. Vielmehr sind die verschiedenen in den Figuren dargestellten Elemente derart wiedergegeben, dass ihre Funktion und genereller Zweck dem Fachmann verständlich wird. In den Figuren dargestellte Verbindungen und Kopplungen zwischen funktionellen Einheiten und Elementen können auch als indirekte Verbindung oder Kopplung implementiert werden. Eine Verbindung oder Kopplung kann drahtgebunden oder drahtlos implementiert sein. Funktionale Einheiten können als Hardware, Software oder eine Kombination aus Hardware und Software implementiert werden.

Nachfolgend werden Techniken im Zusammenhang mit dem Betreiben von Operationsmikroskopiesystemen beschrieben. Die beschriebenen Techniken ermöglichen es, eine automatische Konfiguration von ein oder mehreren Komponenten des Operationsmikroskopiesystems durchzuführen. Derart kann das Operationsmikroskopiesystem in einen Zustand gebracht werden, in dem ein bestimmtes Objekt, z.B. ein Operationsinstrument, geeignet abgebildet wird. Es kann z.B. eine Auto-Positionierung und/oder eine Auto-Zentrierung und/oder eine Auto-Orientierung und/oder eine Auto-Fokussierung und/oder ein Auto-Zoom in Bezug auf einen Referenzpunkt erfolgen, der in Bezug auf ein oder mehrere Objekte definiert ist. Insbesondere werden Techniken offenbart, wie eine automatische Konfiguration des Operationsmikroskopiesystems zur Abbildung eines beweglichen Objekts erfolgen kann.

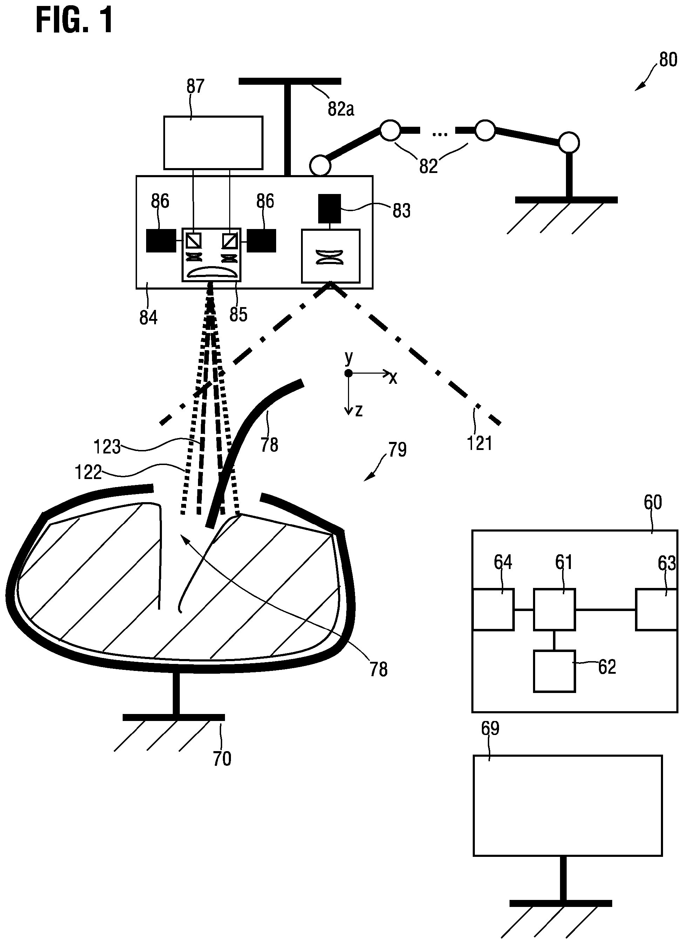

FIG. 1 illustriert schematisch Aspekte in Bezug auf ein beispielhaftes Operationsmikroskopiesystem 80. Das Operationsmikroskopiesystem 80 dient zur mikroskopischen Abbildung eines Untersuchungsbereichs bei einem chirurgischen Eingriff. Dazu wird ein Patient 79 auf einem Operationstisch 70 platziert. Gezeigt ist der Situs 78 mit einem Operationsinstrument 78.

Das Operationsmikroskopiesystem 80 beinhaltet ein robotisches Stativ 82, welches ein positionierbares Kopfteil 81 trägt. Das robotische Stativ 82 kann, je nach Variante, unterschiedliche Freiheitsgrade aufweisen. Es sind robotische Stative 82 bekannt, die sechs Freiheitsgrade für die Positionierung des Kopfteils 81 aufweisen, d.h. Translation entlang jeder der x-Achse, y-Achse und z-Achse und Rotation um jede der x-Achse, y-Achse und z-Achse. Das robotische Stativ 82 kann, wie in FIG. 1 gezeigt, einen Handgriff 82a aufweisen.

Es ist nicht in allen Varianten erforderlich, dass das Stativ 82 robotisch ausgebildet ist. Es wäre auch denkbar, dass das Stativ nur manuell bedient werden kann.

Der Kopfteil 81 umfasst ein Mikroskop 84 auf, welches Optikkomponenten 85, wie beispielsweise eine Beleuchtungsoptik, eine Objektivoptik, eine Zoomoptik, usw. (in FIG. 1 nur schematisch illustriert).

Außerdem umfasst das Mikroskop 84 im dargestellten Beispiel eine Mikroskopkamera 86 (hier eine Stereokamera mit zwei Kanälen; es wäre aber auch eine Mono-Optik denkbar), mit der Bilder des Untersuchungsbereichs erfasst werden können und zum Beispiel auf einem Bildschirm 69 wiedergegeben werden können. Deshalb wird das Mikroskop 84 auch als digitales Mikroskop bezeichnet. Gezeigt ist auch ein Gesichtsfeld 123 der Mikroskopkamera 86.

Im Beispiel der FIG. 1 umfasst das Mikroskop 84 auch ein Okular 87 mit einem zugehörigen Gesichtsfeld 122. Das Okular 87 ist optional. Beispielsweise kann der Detektionsstrahlengang mittels eines Strahlteilers (in FIG. 1 angedeutet) geteilt werden, so dass sowohl ein Bild durch die Kamera 86 erfasst werden kann, wie auch eine Betrachtung durch das Okular 87 möglich ist. Es ist nicht in allen Varianten erforderlich, dass das Mikroskop 84 ein Okular aufweist. Es sind auch rein digitale Mikroskope 84 möglich, bei denen kein Okular vorhanden ist.

Im Beispiel der FIG. 1 umfasst das vom Stativ 82 getragene Kopfteil 81 des Operationsmikroskopiesystems 80 auch eine Umfeldkamera 83. Die Umfeldkamera 83 ist optional. Während die Umfeldkamera 83 im Beispiel der FIG. 1 in das Mikroskop 84 integriert dargestellt sind, wäre es möglich, dass diese getrennt vom Mikroskop 84 angeordnet ist. Die Umfeldkamera kann z.B. eine CCD-Kamera sein. Die Umfeldkamera kann auch eine Tiefenauflösung aufweisen. Alternativ oder zusätzlich zur Umfeldkamera wäre es auch denkbar, das andere assistierende Sensoren vorhanden sind, z.B. ein Abstandssensor (etwa eine Lichtlaufzeitkamera oder einen Ultraschallsensor oder einen Sensor mit strukturierter Beleuchtung). Ein Gesichtsfeld 121 der Umfeldkamera 83 ist in FIG. 1 gezeigt.

Der Chirurg hat somit mehrere Möglichkeiten, den Untersuchungsbereich zu betrachten: Über das Okular 87, über das Mikroskopbild, das von der Kamera 86 aufgenommen wird, oder über ein Übersichtsbild, das von einer Umgebungskamera 83 aufgenommen wird. Der Operateur kann den Untersuchungsbereich auch direkt (ohne Vergrößerung) betrachten.

Die verschiedenen Komponenten des Operationsmikroskopiesystems 80 wie beispielsweise das robotische Stativ 82, das Mikroskop 84 oder die ein oder mehreren weiteren Komponenten wie z.B. die Umfeldkamera 83 werden von einem Prozessor 61 einer Datenverarbeitungseinrichtung 60 gesteuert.

Der Prozessor 61 kann beispielsweise als allgemeiner Zentralprozessor (CPU) und/oder als feldprogrammierbarer Logikbaustein (FPGA) und/oder als anwendungsspezifischer integrierter Schaltkreis (ASIC) ausgebildet sein. Der Prozessor 61 kann Programmcode aus einem Speicher 62 laden und ausführen.

Der Prozessor 61 kann mit verschiedenen Komponenten des Operationsmikroskopiesystems 80 über eine Kommunikationsschnittstelle 64 kommunizieren. Beispielsweise kann der Prozessor 61 das Stativ 82 ansteuern, um das Kopfteil 81 in Bezug auf den Operationstisch 70 zu bewegen, beispielsweise translatorisch und/oder rotatorisch. Der Prozessor 71 kann zum Beispiel Optikkomponenten 85 des Mikroskops 84 ansteuern, um einen Zoom und/oder einen Autofokus zu implementieren. Es könnten Bilder der Umfeldkamera, sofern vorhanden, ausgelesen und ausgewertet werden.

Der Prozessor kann eine Abfolge von mittels einer Kamera des Operationsmikroskopiesystems erfassten Bildern auswerten, um ein Objekt zu verfolgen (tracking). Dazu können zum Beispiel maschinengelernte Algorithmen angewendet werden, die eine Detektion oder optional eine Lokalisierung eines entsprechenden Objekts in den verschiedenen Bildern bereitstellen. Alternativ oder zusätzlich wäre es denkbar, den optischen Fluss basierend auf einer Abfolge von Bildern zu bestimmen; Bereiche, in denen der optische Fluss große Werte annimmt, sind Bereiche, an denen mit größerer Wahrscheinlichkeit ein bewegtes Objekt lokalisiert ist. Entsprechende Techniken sind im Grundsatz im Stand der Technik bekannt (siehe z.B. DE 10 2021 101 694 A1); die konkrete algorithmische Implementierung, die zur Verfolgung eines Objekts verwendet wird, ist nicht entscheidend für die hierin beschriebenen Techniken.

Ferner umfasst die Datenverarbeitungseinrichtung 60 eine Benutzerschnittstelle 63. Über die Benutzerschnittstelle 63 können Befehle eines Chirurgen oder allgemein eines Benutzers des Operationsmikroskopsystems 80 empfangen werden. Die Benutzerschnittstelle 63 kann verschiedene Konfigurationen aufweisen. Beispielsweise kann die Benutzerschnittstelle 63 eine oder mehrere der folgenden Komponenten umfassen: Griffe am Kopfteil 81; Fußschalter; Spracheingabe; Eingabe über eine graphische Benutzeroberfläche; etc.. Es wäre möglich, dass die Benutzerschnittstelle 63 eine graphische Interaktion über Menüs und Knöpfe auf dem Monitor 69 bereitstellt.

Nachfolgend werden Techniken beschrieben, wie es mittels des Operationsmikroskopiesystems 80 möglich ist, eine automatische Konfiguration zur Abbildung eines vordefinierten Objekts - wie beispielsweise einem Operationsinstrument - zu ermöglichen. Eine solche Konfiguration kann z.B. eine Ausrichtung des Mikroskops am Operationsinstrument umfassen. Zum Beispiel kann eine laterale Ausrichtung an einem Operationsinstrument erfolgen, d.h. in X-Richtung und/oder Y-Richtung. Die Ausrichtung kann zum Beispiel eine Zentrierung in Bezug auf einen Referenzpunkt des Objekts umfassen. Beispielsweise könnte eine Zentrierung auf eine Spitze eines Operationsinstruments ausgelöst werden. Alternativ oder zusätzlich zu einer solchen lateralen Ausrichtung einem Objekt kann auch eine Einstellung des Fokus in Bezug auf das Objekt erfolgen. Beispielsweise kann dazu eine Objektivoptik des Mikroskops 84 angesteuert werden, um eine Linse zu verfahren. Es können auch Mikroskope mit fester Brennweite verwendet werden: in einem solchen Fall kann die Z-Position des Mikroskops 84 durch das robotisches Stativ 82 verändert werden. Alternativ oder zusätzlich ist es auch möglich, das Kopfteil 81 und damit das Mikroskop 84 und insbesondere eine Zentralachse der Optikkomponenten 85 zur Abbildung eines bestimmten Objekts zu verkippen (Auto-Orientierung). Eine Pivotierung ist möglich. Eine solche Technik kann den Vorteil aufweisen, dass eine bestimmte Pose des Mikroskops 84 auf das Objekt ermöglicht wird, das heißt eine bestimmte Ausrichtung und Orientierung. Derart können zum Beispiel tiefe Kanäle, die bei einem chirurgischen Eingriff auftreten (cf. Situs 78 in FIG. 1), abgebildet werden. Allgemein kann eine solche automatische Konfiguration zur Abbildung eines vordefinierten Objekts als Auto-XYZ-Konfiguration bezeichnet werden; diese Bezeichnung indiziert, dass die XY-Ausdehnung und Z-Konfiguration des erfassten Bilds eingestellt wird. Auto-XYZ kann z.B. eine Auto-Positionierung (insb. eine Auto-Zentrierung) und/oder Auto-Fokussierung und/oder Auto-Orientierung und/oder Auto-Zoom bezeichnen.

Zum Zwecke der Auto-XYZ-Konfiguration wird eine Soll-Konfiguration von ein oder mehreren Komponenten des Operationsmikroskopiesystems 80 bestimmt und anschließend die ein oder mehreren Komponenten angesteuert, um von der Ist-Konfiguration in die Soll-Konfiguration überführt zu werden.

Die Auto-XYZ-Konfiguration wird in verschiedenen Beispielen durch einen Benutzerbefehl ausgelöst. Es wurden Szenarien beobachtet, bei denen der Benutzerbefehl empfangen wird und sich dann die Szene ändert. Das bedeutet, das ein oder mehrere Objekte bewegt werden; und dann eine andere Position und/oder Orientierung aufweisen, als zum Zeitpunkt des Auslösen des Benutzerbefehls. Der Benutzerbefehl wurde also in einem ersten Zustand der Szene ausgelöst oder abgeben; während dann die Szene einen zweiten Zustand annimmt. Dann kann es in Referenzimplementierungen zu einem ungewünschten Systemverhalten kommen, weil beispielsweise die Auto-Zentrierung auf die alte oder neue Position des Objekts erfolgt, der Benutzer aber gerade das Gegenteil erwartet. Das bedeutet in anderen Worten, dass aufgrund der Änderung der zugrundeliegenden Szene das Systemverhalten und Benutzererwartung diskrepant werden.

Nachfolgend werden Techniken beschrieben, die es im Zusammenhang mit einer durch einen Benutzerbefehl ausgelösten Auto-XYZ-Konfiguration ermöglichen, die Benutzererwartung besser als in Referenzimplementierungen zu erfüllen. Ein deterministischen Systemverhalten wird ermöglicht, welches in unterschiedlichsten Situationen und/oder in Anbetracht unterschiedlichster und auch dynamischer Szenen reproduzierbare und nachvollziehbare Ergebnisse liefert. Solche Techniken beruhen auf der Erkenntnis, dass es insbesondere in Hoch-Stress-Situationen unter Zeitdruck, wie sie typischerweise in einer chirurgischen Umgebung auftreten, notwendig ist, dass das Systemverhalten einer automatischen Steuerung genau mit der Benutzererwartung übereinstimmt.

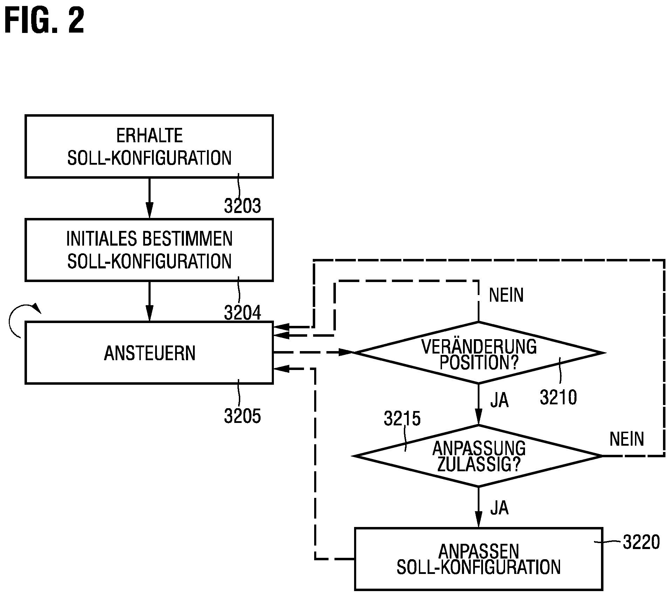

FIG. 2 ist ein Flussdiagramm eines beispielhaften Verfahrens. Das Verfahren der FIG. 2 betrifft Techniken, um eine Assistenzfunktionalität auszuführen. Die Assistenzfunktionalität beinhaltet oder besteht aus einer Auto-XYZ-Konfiguration des Operationsmikroskopiesystems.

Insbesondere betrifft FIG. 2 Techniken, um die Assistenzfunktionalität in Bezug auf bewegte Objekte auszuführen. Beispiele für bewegte Objekte sind insbesondere Operationsinstrumente, die von einem Chirurgen geführt werden.

Beispiele für Operationsinstrumente sind allgemein: Skalpell, Pinzette, Schere, Nadelhalter, Klemme, Sauger, Trokar, Coagulator, Elektrokauter, Retraktor, Bohrer, Spreizer, Osteotom, Nahtmaterial, Knotenschieber, Raspatorium, Hämostat, Lanzette, Drainage, Fadenschneider, Spatel, Ultraschallaspiratoren.

In Box 3203 wird ein Benutzerbefehl empfangen. Der Benutzerbefehl fordert beispielsweise die Konfiguration der Mikroskopkamera zur Bildgebung eines vorgegebenen Objekts, beispielsweise eines Operationsinstruments, implizit oder explizit an. Der Benutzerbefehl kann eine bestimmte Assistenzfunktionalität anfordern. Der Benutzerbefehl wird von einer Benutzerschnittstelle empfangen (vgl. FIG. 1). Der Benutzerbefehl kann zum Beispiel eine Auto-Ausrichtung oder eine Auto-Fokussierung anfordern. Der Benutzerbefehl kann eine Auto-XYZ-Konfiguration auslösen.

In Box 3204 wird eine Soll-Konfiguration des Operationsmikroskopiesystems initial bestimmt. Diese wird basierend auf einem Benutzerbefehl aus Box 3203 bestimmt. Die Soll-Konfiguration kann für die gegenwärtig mittels einer Kamera des Operationsmikroskopiesystems abgebildete Szene mit ein oder mehreren Objekten bestimmt werden. Diese Soll-Konfiguration bezieht sich auf ein oder mehrere Komponenten des Operationsmikroskopiesystems. Beispielsweise kann die Soll-Konfiguration angeben, wie das robotische Stativ das Kopfteil anordnen und/oder orientieren soll. Es könnten auch ein oder mehrere Einstellungen für das Mikroskop angegeben werden, beispielsweise eine zu verwendende Vergrößerung eines Zoomlinsenoptiksystems oder eine Einstellung für den Auto-Fokus und/oder zu verwendende Filter usw.

Dann erfolgt in Box 3205 das Ansteuern von mindestens einer Komponente des Operationsmikroskopiesystems zum Erreichen der Soll-Konfiguration. Das Ansteuern in Box 3205 kann je nach Implementierungsvarianten unterschiedliche Formen annehmen. Beispielsweise könnte Box 3205 das Übergeben eines Einstellungswerts an eine Steuerungs-Hardware des Operationsmikroskopiesystems umfassen. Die Steuerungs-Hardware kann dann diesen Einstellungswert übersetzen in eine Abfolge von konkreten Steuerbefehlen, beispielsweise Analogwerten. Derart wird eine Ist-Konfiguration des Operationsmikroskopiesystems nach und nach verändert in die Soll-Konfiguration. Es wäre aber auch denkbar, dass in Box 3205 eine Abfolge von entsprechenden Einstellungswerten bestimmt wird und direkt an die entsprechenden ein oder mehreren Komponenten übergeben wird.

In Box 3205 können, je nach Assistenzfunktionalität, unterschiedliche Komponenten gesteuert werden. Beispielsweise könnte eine automatische Zentrierung des Gesichtsfelds des Mikroskops auf ein vorgegebenes Objekt erfolgen. Eine solche Zentrierung kann in zwei Dimensionen erfolgen, das heißt unter Verwendung einer XY-Translation des Kopfteils. In einem solchen Fall kann das robotische Stativ angesteuert werden, um eine solche Translation umzusetzen. Alternativ oder zusätzlich wäre es auch denkbar, dass eine automatische Zentrierung in sechs Dimensionen erfolgt, das heißt unter Verwendung von Rotation und Translation des Kopfteils, um beispielsweise in einen Situs etc. zu blicken. Es könnte alternativ oder zusätzlich auch eine Auto-Fokussierung auf ein bestimmtes vorgegebenes Objekt erfolgen. In einem solchen Fall ist es möglich, dass eine bewegliche Optik eines Objektivs des Mikroskops angesteuert wird. In einem solchen Beispiel wäre es aber auch möglich - insbesondere, wenn die Optikkomponenten mit einer festen Brennweite ausgestattet ist - eine Z-Positionierung des Kopfteils durchzuführen. In einem weiteren Szenario wäre es denkbar, dass für eine Menge von Objekten (z.B. solche Objekte, die als relevant erkannt werden) eine Einstellung des Gesichtsfelds durch Anpassen eines Zoomfaktors erfolgt. Beispielsweise könnte der Zoomfaktor so groß wie möglich gewählt werden, sodass alle ausgewählten Objekte noch sichtbar sind (dann braucht es kein robotisches Stativ, weil nur Optik bewegt wird). Manche Objekte können dann nahe beim Rand des gezoomten Gesichtsfelds angeordnet sein.

Das Ansteuern in Box 3205 bewirkt, dass die ein oder mehreren Komponenten des Operationsmikroskopiesystems verstellt werden (zum Erreichen der Soll-Konfiguration). Die Verstellung der ein oder mehreren Komponenten erfolgt nicht instantan, sondern benötigt einen gewissen Zeitraum (d.h. weist eine gewisse Latenz auf). Grund hierfür sind zum Beispiel Limitierung von Aktuatoren, beispielsweise von Stellmotoren (Beschleunigung, maximale Geschwindigkeit). Weitere Gründe sind die Massenträgheit und die notwendige Positioniergenauigkeit, welche eine langsame Verstellung bedingen kann. Beispielsweise kann es ferner bei exponierten Bauteilen - insbesondere dem robotischen Stativ, welches das Kopfteil trägt und das Kopfteil im Operationsraum bewegt - erforderlich sein, die Geschwindigkeit der Bewegung zu begrenzen, um eine Gefährdung des umstehenden Personals durch Kollision zu vermeiden. Typische Zeitdauern zum Bewegen des robotischen Stativs in eine bestimmte Position sind in der Zeitskala von einigen Sekunden bis einigen zehn Sekunden. Auch die Auto-Fokussierung durch Verstellen von Linsen eines Objektivs benötigt eine gewisse Zeit, typischerweise bis zu 1 Sekunde oder 2 Sekunden. Entsprechendes gilt für eine Zoom-Funktionalität.

Verschiedene Beispiele beruhen auf der Erkenntnis, dass es auch aufgrund einer solchen Latenz in der Umsetzung einer bestimmten Konfiguration des Operationsmikroskopiesystems passieren kann, dass sich noch während der Einstellungszeitdauer (oder auch kurz nach vollendeter Einstellung) die der Soll-Konfiguration aus Box 3204 zugrundeliegende Szene verändert. Das bedeutet konkret, dass sich das bewegte Objekt von derjenigen Referenzposition (z.B. im Zentrum des Gesichtsfelds der Kamera) wegbewegen kann, die als Grundlage für die Bestimmung der Soll-Konfiguration diente. Damit ist die Soll-Konfiguration aus Box 3204 veraltet. Dies liegt - allgemein formuliert - daran, dass die Zeitskalen, auf denen einerseits eine Bewegung der bewegten Objekte stattfindet und auf denen andererseits die Verstellung von ein oder mehreren Komponenten des Operationsmikroskopiesystems zum Erreichen der Soll-Konfiguration stattfindet, vergleichbar sind.

Entsprechend wird in Box 3210 überprüft, ob sich die Position des beweglichen Objekts verändert. Dies kann z.B. während oder im zeitlichen Zusammenhang mit der Ansteuerung in Box 3205 erfolgen.

Zum Beispiel kann bei der Überprüfung in Box 3210 eine bestimmte Toleranz akzeptiert werden, sodass geringe Bewegungen ignoriert werden (hochfrequentes Nachjustieren wird derart vermieden).

Wird keine (signifikante) Bewegung in Box 3210 erkannt, so erfolgt keine Anpassung der Soll-Konfiguration, die der Ansteuerung in Box 3205 zugrunde liegt ("Nein"-Pfad aus Box 3210).

Wenn sich die Position des beweglichen Objekts (signifikant) verändert hat ("Ja"-Pfad aus Box 3210), wird Box 3215 ausgeführt. In Box 3215 wird bestimmt, ob sich die Position und/oder Orientierung des beweglichen Objekts innerhalb einer vorgegebenen zeitlichen Schranke und/oder räumlichen Schranke verändert hat. Es wird also überprüft, ob eine Anpassung der Soll-Konfiguration zulässig ist, weil die Position und/oder Orientierung des beweglichen Objekts nur innerhalb der zeitlichen Schranke und/oder räumlichen Schranke verändert wird. Besonders große Änderungen und/oder Änderungen, die in keinem besonders engem zeitlichen Kontext mit der Ansteuerung in Box 3205 stehen, können so vermieden werden. Andererseits werden manche Bewegungen, die in einem genügend engen räumlich und/oder zeitlichen Kontext stehen, bei Ansteuerung berücksichtigt: Wenn die Anpassung zulässig ist, wird in Box 3220 die Soll-Konfiguration angepasst und das Ansteuern in Box 3205 berücksichtigt dann die angepasste Soll-Konfiguration.

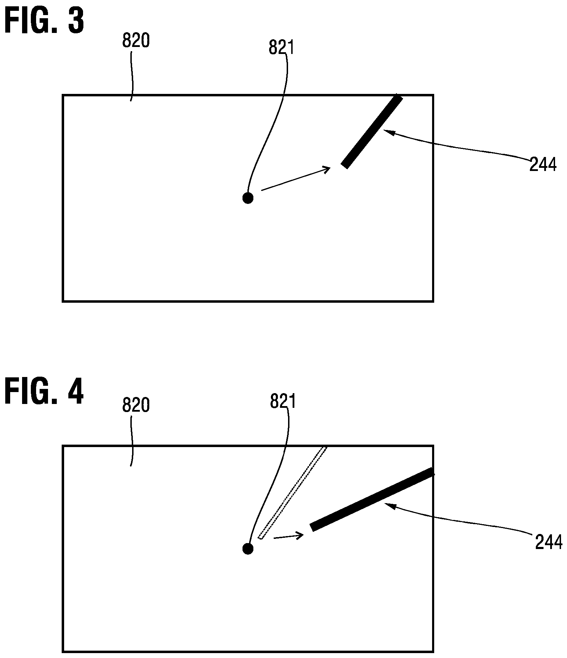

Als nächstes werden die Effekte eines solchen Verfahrens anhand eines konkreten Beispiels diskutiert. Dies erfolgt in Abgrenzung zu Referenzimplementierungen. In FIG. 3 ist der initiale Zustand gezeigt. Ein Operationsinstrument 244 ist gegenüber dem Zentrum 821 des Mikroskopbilds 820 versetzt angeordnet. Es wird dann ein Auto-Zentrierungsbefehl erhalten (laterale XY-Zentrierung); der Befehl fordert an, dass die Spitze des Operationsinstruments 244 im Zentrum angeordnet wird. Das ist durch den Pfeil in FIG. 3 illustriert. Während das Kopfteil bewegt wird, um diese Auto-Zentrierung umzusetzen, wird auch das Operationsinstrument 244 bewegt, vgl. FIG. 4, dort der Pfeil. Das bedeutet also, dass sich die der Auto-Zentrierung zugrundeliegende Szene noch während der Bewegung des Kopfteils verändert. Dies bewirkt, dass (ohne weitere Maßnahmen zur Anpassung der Soll-Konfiguration, also gemäß Referenzimplementierungen, z.B. dem in US 10,456,035 B2 beschriebenen "spezifizierten Zeitkorrekturmodus") das Zentrum 126 nach Beendigung des Positionierungsvorgangs nicht koinzident mit der Spitze des Operationsinstruments 244 wäre. Dies kann, zumindest unter bestimmten Voraussetzungen, dazu führen, dass die Benutzererwartung (etwa: "auf Toolspitze zentriert") nicht erfüllt wird. Im Beispiel der FIG. 3 und der FIG. 4 erfolgt also eine einmalige Zentrierung: nach Aktivierung der assistierenden Funktion (beispielhaft über einen Sprachbefehl oder einen Taster eines Fußschaltpultes) zentriert sich das Kopfteil auf die zum Zeitpunkt der Aktivierung vorliegenden Operationsinstrument-Aktivität. Da in der Regel der Chirurg seine Arbeit fortsetzt, während das Operationsmikroskop noch seine Bewegung durchführt, führt dies dazu, dass nach der Bewegung des Operationsmikroskops das Zentrum des Gesichtsfelds 123 des Mikroskops 84 nicht mit der Operationsinstrument-Position übereinstimmt. Für eine korrekte Zentrierung müsste die einmalige Zentrierung erneut aktiviert und die Operationsinstrument-Aktivität pausiert werden. Dies ist jedoch unvorteilhaft, da es zu einer Unterbrechung des OP-Workflows und ggf. der Konzentration des Chirurgen führt. Entsprechend wäre es in einem anderen (nachteiligen) Referenzszenario (z.B. dem in US 10,456,035 B2 beschriebenen "bedingten Automatikmodus") auch denkbar, dass der die Bewegung des Operationsinstruments244 kontinuierlich gefolgt wird. Es erfolgt also eine kontinuierliche Zentrierung auf die Aktivität des Operationsinstruments, d.h. nach Aktivierung der assistierenden Funktion (beispielhaft über einen Sprachbefehl oder einen Taster eines Fußschaltpultes) wird das Mikroskop kontinuierlich auf die Aktivität zentriert, bis die assistierende Funktion wieder deaktiviert wird. Diese Referenzimplementierung wird jedoch oftmals als störend erachtet, da nicht von gewollten und ungewollten Bewegungen der Objekte unterschieden werden kann und folglich das Operationsmikroskop Bewegungen durchführen würde, die störend auf die Konzentration und Fokussierung des Chirurgen wären (und in Konsequenz auch das Risiko für den Patienten erhöhen würden).

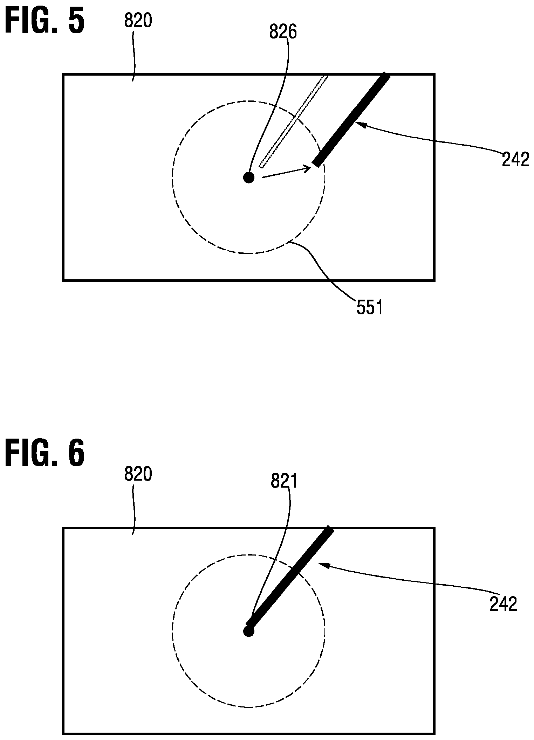

Solche Probleme der voranstehend geschilderten Referenzszenarien werden durch die Berücksichtigung der zeitlichen und/oder räumlichen Schranke in Box 3215 zumindest gelindert. Bewegungen des Objekts führen nur innerhalb der zeitlichen Schranke und/oder räumlichen Schranke zu einer Anpassung der Soll-Konfiguration. Das ist in FIG. 5 gezeigt.

FIG. 5 illustriert Aspekte in Bezug auf eine räumliche Schranke 551. Die räumliche Schranke ist im illustrierten Beispiel im Zusammenhang mit der initial basierend auf dem Benutzerbefehl bestimmten Soll-Konfiguration (vgl. Box 3204) bestimmt. Im Detail: Mittels der initialen Soll-Konfiguration soll das vorgegebene Objekt, hier die Spitze des Operationsinstruments, im Zentrum des Gesichtsfelds 123 der Mikroskopkamera und damit im Zentrum des Mikroskopbilds positioniert werden. Das definiert eine Referenzposition des vorgegebenen Objekts; im vorliegenden Beispiel ist diese Referenzposition im Zentrum des Mikroskopbilds. Die räumliche Schranke ist als maximaler Abstand in Bezug auf mit der initialen Soll-Konfiguration assoziierte Referenzposition festgelegt.

Im Beispiel der FIG. 5 bewegt sich die Spitze 242 (illustriert durch den Pfeil) von dieser Referenzposition weg; und zwar um einen Abstand, der kleiner ist als die räumliche Schranke 551. Deshalb wird die (initial basierend auf dem Benutzerbefehl bestimmte) Soll-Konfiguration angepasst, so dass das Zentrum des Gesichtsfelds dem bewegten Operationsinstrument 242 folgt und letztendlich die bewegte Spitze 242 im Zentrum des Mikroskopbilds 820 angeordnet ist, vergleiche FIG. 6. Wäre die Bewegung der Spitze 242 größer als die räumliche Schranke, so kann ein Fehler ausgegebenen werden und/oder die Auto-Zentrierung abgebrochen werden ("Nein"-Pfad aus Box 3125 in FIG. 2).

Voranstehend wurde ein Szenario beschrieben, bei dem die räumliche Schranke 551 basierend auf einer Referenzposition des Objekts bestimmt wird, die mit der initial basierend auf dem Benutzerbefehl bestimmten Soll-Konfiguration assoziiert ist. Es wäre auch denkbar, dass nach einer Umpositionierung des Operationsinstruments 242 innerhalb der räumlichen Schranke 551 die räumliche Schranke 551 wiederum angepasst wird. Die räumliche Schranke 551 kann also "nachgezogen" werden. Das bedeutet, dass die Referenzposition angepasst werden kann, in Übereinstimmung mit der entsprechend angepassten Soll-Konfiguration. Eine solche iterative Anpassung der räumlichen Schranke 551 kann so lange fortgesetzt werden, bis die Ist-Position und die Soll-Position des Operationsinstruments 242 stabil konvergieren. Dies entspricht in etwa der Funktion eines Tiefpassfilters: kleinere Bewegungen des Operationsinstruments 242 bewirken ein "Nachziehen" der Auto-Zentrierung, während größere Bewegungen des Operationsinstruments 242 keine Anpassung der Auto-Zentrierung bewirken.

Eine räumliche Schranke wie in FIG. 5 und FIG. 6 diskutiert kann kombiniert werden mit einer zeitlichen Schranke. Die zeitliche Schranke kann aber auch ohne räumliche Schranke verwendet werden. Aspekte mit der zeitlichen Schranke werden als nächstes diskutiert.

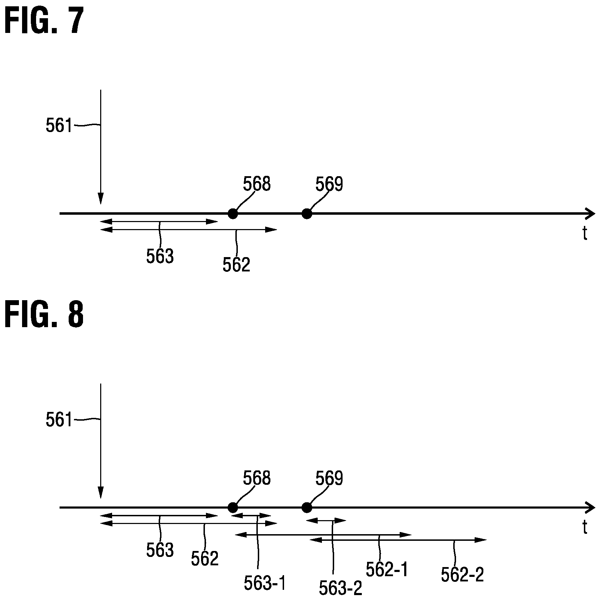

FIG. 7 illustriert Aspekte in Bezug auf eine zeitliche Schranke 562. Die zeitliche Schranke 562 gibt einen Zeitraum an, innerhalb dessen Bewegungen des vorgegebenen Objekts, welches die Grundlage für die Soll-Konfiguration des Operationsmikroskopiesystems bildet, potentiell berücksichtigt werden, um die Soll-Konfiguration anzupassen. Wenn also eine Bewegung innerhalb der zeitlichen Schranke stattfindet, wird diese berücksichtigt (z.B. sofern die Bewegung auch innerhalb der räumlichen Schranke liegt); ansonsten nicht.

In FIG. 7 wird ein Benutzerbefehl 561 empfangen (der z.B. eine Auto-Zentrierung anfordert, sodass die Soll-Konfiguration initial bestimmt werden kann, vgl. Box 3204) und dann erfolgt in einem Zeitraum 563 das Verstellen von ein oder mehreren Komponenten des Operationsmikroskopiesystems. Dieser Zeitraum kann z.B. von einer Trajektorie abhängig sein, die das robotische Stativ abfährt, um das Kopfteil geeignet zu positionieren.

Die zeitliche Schranke 562 weist im Beispiel der FIG. 7 eine Länge auf, die 150 % des Zeitraums 563 beträgt. Das heißt, die zeitliche Schranke 563 ist durch die Zeitdauer zum Erreichen der initial basierend auf dem Benutzerbefehl 561 bestimmten Soll-Konfiguration bestimmt.

Innerhalb der zeitlichen Schranke 562 erfolgt eine Bewegung 568 des Objekts; beispielsweise wird das Operationsinstrument bewegt; diese Bewegung wird - weil sie innerhalb der zeitlichen Schranke stattfindet - zum Anpassen der Soll-Konfiguration berücksichtigt. Eine spätere Bewegung 569 liegt aber außerhalb der zeitlichen Schranke 562 und wird daher nicht zum Anpassen der Soll-Konfiguration berücksichtig.

Im Beispiel der FIG. 7 ist die zeitliche Schranke 562 initial festgelegt, nämlich durch die Dauer der Zeitspanne 563, die basierend auf der initial basierend auf dem Benutzerbefehl bestimmten Trajektorie zum Erreichen der Soll-Konfiguration ermittelt wird. Es wäre aber auch möglich, dass die zeitliche Schranke dynamisch angepasst wird, was in FIG. 8 gezeigt ist.

FIG. 8 illustriert Aspekte in Bezug auf eine mehrfach angepasste zeitliche Schranke 562, 562-1, 562-2. Die Schranke 562, 562-1, 562-2 wird so lange angepasst (also "nachgezogen"), bis die Ist-Konfiguration stabil der Soll-Konfiguration entspricht, d.h. so lange bis die Soll-Konfiguration endgültig erreicht wird. Im Beispiel der FIG. 7 löst die Bewegung 568 (so wie im Zusammenhang mit FIG. 7 diskutiert) eine Anpassung der Soll-Konfiguration aus (die Soll-Position ist zu diesem Zeitpunkt noch nicht endgültig erreicht); deshalb erfolgt eine erneute Ansteuerung des robotischen Stativ z.B. zum Re-Zentrieren des Gesichtsfelds; das robotische Stativ wird während des Zeitraums 563-1 angesteuert. Die erneute Bewegung 568 löst im Beispiel der FIG. 8 eine Re-Initialisierung der zeitlichen Schranke 562-1 aus, sodass nunmehr auch die Bewegung 569 berücksichtigt wird. Die Bewegung 569 löst wieder eine Bewegung aus (Zeitraum 563-2), was wiederum eine Re-Initialisierung der zeitlichen Schranke 562-2 auslöst. Dann ist die Ist-Konfiguration stabil gleich der Soll-Konfiguration und es erfolgt keine Re-Initialisierung der zeitlichen Schranke mehr. Das Objekt ist dann z.B. endgültig und stabil im Zentrum des Mikroskopbilds angeordnet.

Während in den Szenarien der FIG. 7 und FIG. 8 Beispiele gezeigt sind, bei denen die zeitliche Schranke 562 jeweils einen Startzeitpunkt aufweist, der mit dem Startzeitpunkt der Einstellung von ein oder mehreren Komponenten im Zeitraum 563 zusammenfällt, wäre es generell auch denkbar, dass die hierin beschriebenen zeitlichen Schranken einen anderen Startzeitpunkt aufweisen. Beispielsweise könnte der Beginn einer zeitlichen Schranke gegenüber dem Empfang des Benutzerbefehls 561 und/oder gegenüber dem Beginn der Bewegung einer oder mehrerer Komponenten des Operationsmikroskopiesystems um eine bestimmte vordefinierte Zeitspanne versetzt sein. Neben einer solchen zeitlichen Verschiebung wäre es auch möglich, die Zeitschranke ereignisgesteuert zu starten. Beispielsweise könnte geprüft werden, ob das Mikroskop um eine bestimmte Strecke bewegt wurde.

Im Zusammenhang mit der Dimensionierung der zeitlichen und/oder räumlichen Schranken: Es wäre möglich, dass deren Größen fest vorgegebene sind. Es wäre aber auch möglich, dass deren Größen dynamisch bestimmt wird, beispielsweise basierend auf einem Typ des Objekts; einer Einstellung von ein oder mehreren Komponenten des Operationsmikroskopiesystems, z.B. Vergrößerung des Mikroskops, Arbeitsabstand, chirurgischer Kontext (z.B. OP-Typ), Lagerung des Patienten, usw. Solche Techniken beruhen auf der Erkenntnis, dass bestimmte Objekttypen typischerweise eine geringere Bewegung aufweisen als andere Objekttypen. Dies bedeutet, dass die Toleranz im Zusammenhang mit der Anpassung der Soll-Konfiguration für solche Objekttypen kleiner gewählt werden kann als für andere Objekttypen.

Mittels der räumlichen Schranke und/oder der zeitlichen Schranke können also verschiedene Szenarien umgesetzt werden. Es werden nachfolgend einige beispielhafte Szenarien diskutiert. In einem ersten Szenario aktiviert der Chirurg einmalig die Auto-Zentrierung auf ein bestimmtes Operationsinstrument. Es wird dann die Position und/oder Orientierung des Mikroskops basierend auf der gegenwärtigen Positionierung des Operationsinstruments relativ in Bezug auf das Mikroskop berechnet, das heißt es wird die Soll-Konfiguration für das robotische Stativ initial bestimmt. In diesem Zusammenhang erfolgt auch die Festlegung einer Fläche oder eines Volumens um diese Zielposition oder die Spitze des Operationsinstruments herum, innerhalb derer die Zielpose während der Fahrt verändert werden darf. Das bedeutet also, dass die räumliche Schranke in Bezug auf eine Referenzposition bestimmt ist, die basierend auf der initialen Soll-Konfiguration bestimmt wird. Dann beginnt die robotische Bewegung in die Soll-Konfiguration. Die Aktivität des Operationsinstruments wird kontinuierlich ausgewertet (tracking). Es erfolgt eine Anpassung der Zielpose, solang sich die Spitze des Operationsinstruments innerhalb der zuvor berechneten Fläche oder des zuvor berechneten Volumens befindet. Bei Erreichen der Zielpose wird die robotische Bewegung beendet und es erfolgt auch keine weitere kontinuierliche Anpassung der Soll-Konfiguration. Dies entspricht also einer räumlichen Schranke, kombiniert mit einer zeitlichen Schranke, die durch das tatsächliche Erreichen der Soll-Position definiert ist. In einem weiteren Szenario wird die einmalige Zentrierung durch den Chirurgen, wie bereits obenstehend erläutert, aktiviert. Es wird dann eine Soll-Konfiguration - z.B. eine Zielpose des robotischen Stativs - bestimmt. Es wird auch eine Zeit festgelegt (zum Beispiel in Abhängigkeit von der geschätzten Fahrtdauer zum Erreichen der Soll-Konfiguration), innerhalb derer die Bewegung des Operationsinstruments zu einer Anpassung der Soll-Konfiguration führt. Die robotische Bewegung wird dann begonnen und die Aktivität des Operationsinstruments wird kontinuierlich ausgewertet (tracking). Die Soll-Konfiguration wird während der voranstehend bestimmten zeitlichen Schranke basierend auf der detektierten Aktivität angepasst. Es sind auch Kombinationen dieser beiden Varianten denkbar.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

Beispielsweise wurden voranstehend verschiedene Aspekte im Zusammenhang mit einer Auto-XYZ-Konfiguration in Bezug auf ein Operationsinstrument beschrieben. Als allgemeine Regel wäre es aber denkbar, dass eine Auto-XYZ-Konfiguration auch auf andere Objekte erfolgt, beispielsweise charakteristische anatomische Merkmale des Patienten.

Ferner wurden voranstehend Aspekte beschrieben, bei denen eine Auto-XYZ-Konfiguration in Bezug auf ein einzelnes Objekt erfolgt. Allgemein wäre es aber denkbar, dass die Auto-XYZ-Konfiguration in Bezug auf mehrere Objekte erfolgt. Beispielsweise kann ein von den Positionen der mehreren Objekte abhängiger Referenzpunkt bestimmt werden (zum Beispiel ein geometrischer Mittelpunkt oder ein Aktivitätszentren), und dann eine Auto-XYZ-Konfiguration in Bezug auf diesen Referenzpunkt erfolgen. Beispielsweise könnte aus allen sichtbaren Objekten eine Teilmenge relevanter Objekte ausgewählt werden, wobei dann die Auto-XYZ-Konfiguration in Bezug auf das mindestens eine Objekt in dieser Teilmenge erfolgt.

Ferner wurden obenstehend verschiedene Aspekte im Zusammenhang mit einem robotischen Stativ beschrieben. Es ist nicht unbedingt notwendig, dass das Operationsmikroskopiesystem ein robotisches Stativ aufweist. Das Operationsmikroskopiesystem könnte auch ein teilweise robotisches Stativ oder ein manuelles Stativ aufweisen.

## Patentansprüche

1. Computer-implementiertes Verfahren zum Steuern eines Operationsmikroskopiesystems (80) mit einem Stativ (82) und einem vom Stativ (82) getragenen Mikroskop (84)
wobei das Verfahren umfasst:
- Empfangen (3203) eines Benutzerbefehls (561), der eine Konfiguration des Operationsmikroskopiesystems (80) zur Bildgebung eines vorgegebenen Objekts (231, 232, 233) anfordert,
- basierend dem Benutzerbefehl, initiales Bestimmen (3204) einer Soll-Konfiguration des Operationsmikroskopiesystems (80),
- Ansteuern (3205) von mindestens einer Komponente (82, 83, 84) des Operationsmikroskopiesystems (80) zum Erreichen der Soll-Konfiguration,
- im zeitlichen Zusammenhang mit dem Ansteuern (3205) der mindestens einen Komponente (82, 83, 84): Bestimmen, ob sich die Position und/oder Orientierung des beweglichen Objekts (231, 232, 233) innerhalb einer vorgegebenen zeitlichen Schranke (562) und/oder innerhalb einer vorgegebenen räumlichen Schranke (551) verändert, und
- wenn sich die Position und/oder Orientierung des beweglichen Objekts (231, 232, 233) innerhalb der vorgegebenen zeitlichen Schranke (562, 562-1, 562-2) und/oder innerhalb der vorgegebenen räumlichen Schranke (551) verändert, Anpassen (3220) der Soll-Konfiguration.

2. Computer-implementiertes Verfahren nach Anspruch 1, wobei das Verfahren weiterhin umfasst:
- Bestimmen einer Referenzposition des vorgegebenen Objekts basierend auf der initial basierend auf dem Benutzerbefehl bestimmten Soll-Konfiguration,
wobei die räumliche Schranke in Bezug auf die Referenzposition bestimmt ist.

3. Computer-implementiertes Verfahren nach Anspruch 2, wobei das Verfahren weiterhin umfasst:
- Anpassen der Referenzposition basierend auf der angepassten Soll-Konfiguration, wenn sich die Position des beweglichen Objekts (231, 232, 233) innerhalb der vorgegebenen zeitlichen Schranke und/oder der vorgegebenen räumlichen Schranke verändert.

4. Computer-implementiertes Verfahren nach einem der voranstehenden Ansprüche, wobei das Verfahren weiterhin umfasst:
- initiales Bestimmen der zeitlichen Schranke (562, 562-1, 562-2) basierend auf einer Zeitdauer (563) zum Erreichen der initial basierend auf dem Benutzerbefehl (561) bestimmten Soll-Konfiguration.

5. Computer-implementiertes Verfahren nach Anspruch 4, wobei das Verfahren weiterhin umfasst:
- Anpassen der initial basierend auf der Zeitdauer (563) bestimmten zeitlichen Schranke (562, 562-1, 562-2) bis zum endgültigen Erreichen der Soll-Konfiguration.

6. Computer-implementiertes Verfahren nach einem der voranstehenden Ansprüche, wobei das Verfahren weiterhin umfasst:
- Bestimmen eines Typs des beweglichen Objekts (231, 232, 233)
wobei die vorgegebene zeitliche und/oder räumliche Schranke vom Typ des vorgegebenen Objekts (231, 232, 233) abhängt.

7. Computer-implementiertes Verfahren nach einem der voranstehenden Ansprüche,
wobei der Benutzerbefehl eine Auto-Zentrierung eines Gesichtsfelds (122, 123) des Mikroskops (84) auf das vorgegebene Objekt (231, 232, 233) anfordert,
wobei die mindestens eine Komponente das zumindest teilweise robotisch ausgebildete Stativ (82) umfasst.

8. Computer-implementiertes Verfahren nach einem der voranstehenden Ansprüche,
wobei der Benutzerbefehl eine Auto-Fokussierung einer Bilderebene des Mikroskops auf das vorgegebene Objekt (231, 232, 233) anfordert,
wobei die mindestens eine Komponente eine Objektivoptik (85) des Mikroskops (84) und/oder das zumindest teilweise robotisch ausgebildete Stativ (82) umfasst.

9. Datenverarbeitungseinrichtung (60) zur Steuerung eines Operationsmikroskopiesystems (80), wobei die Datenverarbeitungseinrichtung (60) einen Prozessor (61) umfasst, der eingerichtet ist, um Programmcode aus einem Speicher (62) zu laden und auszuführen, wobei das Ausführen des Programmcodes bewirkt, dass der Prozessor (61) die folgenden Schritte ausführt:
- Empfangen (3203) eines Benutzerbefehls (561), der eine Konfiguration des Operationsmikroskopiesystems (80) zur Bildgebung eines vorgegebenen Objekts (231, 232, 233) anfordert,
- basierend dem Benutzerbefehl, initiales Bestimmen (3204) einer Soll-Konfiguration des Operationsmikroskopiesystems (80),
- Ansteuern (3205) von mindestens einer Komponente (82, 83, 84) des Operationsmikroskopiesystems (80) zum Erreichen der Soll-Konfiguration,
- im zeitlichen Zusammenhang mit dem Ansteuern (3205) der mindestens einen Komponente (82, 83, 84): Bestimmen, ob sich die Position und/oder Orientierung des beweglichen Objekts (231, 232, 233) innerhalb einer vorgegebenen zeitlichen Schranke (562) und/oder innerhalb einer vorgegebenen räumlichen Schranke (551) verändert, und
- wenn sich die Position und/oder Orientierung des beweglichen Objekts (231, 232, 233) innerhalb der vorgegebenen zeitlichen Schranke (562, 562-1, 562-2) und/oder innerhalb der vorgegebenen räumlichen Schranke (551) verändert, Anpassen (3220) der Soll-Konfiguration.

10. Datenverarbeitungseinrichtung (60) nach Anspruch 9, wobei der Prozessor (61) eingerichtet ist, um das Verfahren nach einem der Ansprüche 1 bis 8 auszuführen.

11. Operationsmikroskopiesystem (80), welches die Datenverarbeitungseinrichtung (60) nach Anspruch 9 oder 10 umfasst.
